# EUROPEAN PATENT SPECIFICATION

(11) **EP 2 701 851 B1**
(45) Date of publication and mention of the grant of the patent: **24.04.2024**
(21) Application number: 12777488.3
(22) Date of filing: 13.03.2012
(51) Int. Cl.: B03C 5/02, B03C 1/02, G01N 15/14, C12Q 1/02, B03C 1/033, B03C 1/035, B03C 1/28, G01N 15/10

(54) **FLUIDIC IN-LINE PARTICLE IMMOBILIZATION AND COLLECTION SYSTEM AND METHOD FOR USING THE SAME**
SAMMELSYSTEM ZUR IMMOBILISIERUNG FLUIDISCHER INLINE-TEILCHEN SOWIE VERFAHREN DAFÜR
SYSTÈME FLUIDIQUE D'IMMOBILISATION ET DE COLLECTE DE PARTICULES EN CONTINU ET LEUR PROCÉDÉ D'UTILISATION

(30) Priority: 29.04.2011 US 201161480811 P
(43) Date of publication of application: 05.03.2014
(73) Proprietor: Becton Dickinson and Company, Franklin Lakes, NJ 07417-1880 (US)
(72) Inventor: CHEN, Yong, San Jose, California 95135 (US)
(74) Representative: Duxbury, Stephen
(86) International application number: PCT/US2012/028948
(87) International publication number: WO 2012/148583

(56) References cited:
- EP-A1- 1 331 035
- WO-A1-94/11078
- JP-A- 2004 502 530
- JP-A- 2007 522 938
- US-A- 5 030 002
- US-A- 5 541 072
- US-A1- 2004 234 898
- US-A1- 2008 269 432
- US-A1- 2009 026 080
- US-A1- 2009 053 799
- US-A1- 2010 228 513
- US-B1- 6 346 196
- US-B1- 6 432 630

## Description

### INTRODUCTION

The disclosure refers to a fluidic sorting system.

Flow-type particle sorting systems, such as sorting flow cytometers, are used to sort particles in a fluid sample based on at least one measured characteristic of the particles. In a flow-type particle sorting system, particles, such as molecules, analyte-bound beads, or individual cells, in a fluid suspension are passed in a stream by a detection region in which particle sensing means senses particles contained in the stream of the type to be sorted. The particle sensing means, upon detecting a particle of the type to be sorted, triggers a sorting mechanism that selectively isolates the particle of interest.

Particle sensing typically is carried out by passing the fluid stream by a detection region in which the particles are exposed to an excitation light, typically from one or more lasers, and the light scattering and fluorescence properties of the particles are measured. Particles or components thereof can be labeled with fluorescent dyes to facilitate detection, and a multiplicity of different particles or components may be simultaneously detected by using spectrally distinct fluorescent dyes to label the different particles or components. Typically, detection is carried out using a multiplicity of photodetectors to facilitate the independent measurement of the fluorescence of each distinct dye.

One widely used type of flow-type particle sorting system is the electrostatic sorting type. In an electrostatic sorter, the fluid suspension is jetted from a nozzle and vibrated to break the stream into uniform discrete drops. The sorting mechanism includes a drop charging means connected to the stream to charge drops containing a particle of the type to be sorted with an electrical charge as it breaks off from the jet stream. The stream of drops is passed through a transverse electrostatic field established by a pair of oppositely charged deflection plates. Uncharged drops are not deflected passing through the electrostatic field and are collected by a central receptacle. Charged drops containing a particle of the type to be sorted are deflected in a direction and amount related to the polarity and magnitude of the drop charge and are collected in a separate collection receptacle. The BD FACSAria^{™} Flow Cytometer from BD Biosciences (San Jose, CA) is a particle sorter of the electrostatic sorting type.

An alternative type of flow-type particle sorter is based on the use of a moving droplet capture tube in a closed fluidic system, such as described in U.S. Patent No. 5,030,002. In this type of sorter, a capture tube (also referred to as a catcher tube) is positioned in the flow stream downstream from the particle sensing means. The catcher tube normally is positioned in the flow stream but outside the particle path, which is focused in the center of the flow stream. The catcher tube is capable of moving transversely within the stream such that it can be moved in and out of the particle path. The particle sensing means, upon detecting a particle of the type to be sorted, triggers movement of the catcher tube into the particle path to catch the particle to be sorted, and then back out of the particle path before the next particle reaches the tube. The BD FACSCalibur^{™} Flow Cytometer with Sorting Option from BD Biosciences (San Jose, CA) is a flow-type particle sorter based on the moving catcher tube sorting mechanism.

In a number of applications in flow cytometry, such as sorting cells for therapeutic use, a closed fluidic system is desirable to avoid contamination of the sample. A disadvantage of electrostatic sorters is that the generation of an in-air particle stream, and the concomitant formation of aerosols, makes it more difficult to isolate the sample to avoid either contamination of the sample or exposure of the operator to a hazardous sample aerosol. The moving catcher tube type of sorter has the advantage that the flow stream is fully closed and not exposed to the air, making it easier to maintain the sterility of the sorting environment. Closed fluidic sorters generally are more cost effective than droplet sorters, and the closed fluidic pathways of these sorters also provide a safe means of analyzing hazardous samples.

One drawback of closed fluidic sorters of the type described in U.S. Patent No. 5,030,002 is that the moving catcher tube is always in the fluid stream, even when out of the particle path, and receiving sheath fluid. As such, cells collected by the catcher tube are often diluted in sheath fluid to the extent that the cell density is incompatible (e.g., too low) for a number of subsequent research and clinical applications. The problem is particularly significant when sorting rare cells. This problem of cell dilution has prevented the widespread adoption of closed fluidic sorters, despite the safety and other advantages that these sorters provide.

Typical flow cytometers that analyze the light scatter and fluorescence properties of cells are not designed to provide subcellular images of the analyzed cells. Imaging Flow Cytometry (IFC) is a technology that combines the photometric analysis provided by a flow cytometer with the morphometric analysis provided by an imaging system. A commercial example of an IFC system is the ImageStream by Amnis, which uses time delay and integration (TDI) CCD technology to capture spectrally resolved images for every cell passing through the laser excitation zone in a hydrodynamically focused flow cell. IFC has reduced sensitivity and throughput relative to flow cytometry, and is therefore difficult to use to study rare events and or cells with weakly staining antigens. Furthermore, following analysis, the cells are disposed into a waste tank and cannot be used for further analysis or for cell culture.

Flow cytometers and sorting flow cytometers are described in Shapiro, 2003, Practical Flow Cytometry (John Wiley and Sons, Inc. Hoboken, NJ); and "Flow Sorters for Biological Cells" by Tore Lindmo, Donald C. Peters, and Richard G. Sweet, Flow Cytometry and Sorting, 2d ed. (New York: Wiley-Liss, Inc., 1990), pages 145-169. Flow cytometers and sorting flow cytometers are commercially available from, for example, BD Biosciences (San Jose, CA).

### SUMMARY

The present invention provides a closed-fluidic sorting system in accordance with claim 1 and a method of use in accordance with claim 8 that enable the capture, concentration and/or analysis of sorted particles.

In one aspect, the system comprises a closed-fluidic sorter with an integrated in-line cell collection mechanism configured to localize sorted particles, such as cells, inside an in-line flow-through chamber that is an optical cuvette, and an imaging system that enables the imaging of the localized sorted particles. The cell collection mechanism is configured to localize the sorted cells and to allow excess fluid (sheath and sample) to pass through the cuvette and be disposed.

Localization of the particles in the cuvette preferably is carried out magnetically. Particles (e.g., cells) are labeled with magnetic particles prior to analysis. The magnetically labeled sorted cells are captured in the cuvette by a magnet positioned adjacent to the flow-through chamber of the cuvette. In a preferred embodiment, the localization of the sorted cells is reversible, such that, after imaging, the sorted cells can be released for further use, such as cell culture, or, optionally, discarded.

The imaging system provides for the analysis of the cells by a microscopy technology, such as, for example, conventional microscopy, fluorescence imaging microscopy, confocal microscopy, or laser scanning microscopy technologies.

In another aspect, the system comprises a flow cytometric closed-fluidic sorter with an integrated in-line cell collection mechanism configured to localize sorted particles, such as cells, inside a flow-through chamber while allowing fluids to pass through. The system enables concentrating the sorted cells inside the flow-through chamber. In a preferred embodiment, the localization of the sorted cells is reversible, such that, after imaging, the sorted cells can be released for further use, such as cell culture, or, optionally, discarded. The released cells will be significantly concentrated relative to the concentration in the sorted sample.

For use in concentrating the sorted particles, localization of the particles in the flow-through chamber can be carried out magnetically or using any other method that retains the particles while allowing fluids (sheath and sample) to flow through the chamber. In one embodiment, the particles are localized using an acoustic focusing trap that uses acoustic waves to hold particles in place. Turning off the acoustic wave source releases the particles from the trap.

The above aspects of the invention can be combined and carried out simultaneously. For example, using a system that comprises a closed-fluidic sorter with an integrated in-line cell collection mechanism configured to reversibly magnetically localize sorted particles inside an in-line optical cuvette (flow-through chamber), and an imaging system that enables the imaging of the localized sorted particles, enables further optical analysis of the sorted particles, concentration of the sorted particles, and recovery of the sorted particles for further use.

The subject systems and methods find use in a variety of different applications where reversibly immobilizing a particle of interest is desired. As noted above, closed fluidic sorting systems of the type described in U.S. Patent No. 5,030,002 sort particles using a movable catcher tube at a location downstream of the interrogation point. When a particle of interest is identified by the optical analysis carried out at the interrogation point, the opening of the catcher tube is moved into the center of the flow stream (i.e., the core stream), into the path of the particle, such that the particle enters the catcher tube. When a particle of interest is not detected at the interrogation point, the opening of the catcher tube is held away from the core stream and only sheath fluid is collected. Accordingly, one drawback of such closed-fluidic sorters is that the particles of interest are collected along with a constant flow of sheath fluid that dilutes the sorted particles. The present invention solves this problem by enabling concentration of the sorted particles. Immobilization of the particles in the flow-through chamber while allowing fluid to pass through concentrates the particles. Reversing the immobilization (e.g., by deactivating the immobilization means) releases the particles from the flow-through chamber and permits their collection at densities suitable for subsequent research and/or clinical applications.

As noted above, fluidic sorters of the invention optionally include an analysis component configured to analyze one or more particles within the flow-through chamber. The ability to analyze the immobilized particles finds use in research applications, where analysis of one or more features of the particle (enabled by the reversible immobilization of the particle in a flow-through chamber in accordance with the invention) leads to a better understanding of the nature of the particle. Such applications also include diagnostic applications, e.g., applications where analysis of one or more features of the particle facilitates diagnosis of a subject with respect to a given condition, e.g., a disease condition.

The ability to analyze the immobilized particles also finds use in a variety of therapeutic applications, e.g., cell therapy applications. For example, analysis of a reversibly immobilized cell in a fluidic sorting system of the invention may facilitate the selection or exclusion of that cell for its subsequent transplantation into a patient with a particular medical condition. By way of example, a cell (e.g., a stem cell, a bone marrow cell, or any other cell that may find use in a therapeutic application) can be reversibly immobilized and analyzed for one or more subcellular and/or extracellular features (e.g., physical, chemical, and/or biological features) indicative or predictive of the efficacy of that cell in a particular cell-based therapeutic application. Such applications include the treatment of a variety of medical conditions including, but not limited to, cancer (e.g., leukemia, lymphoma, and cancers of the breast, pancreas, lung, kidney, nervous system (e.g., brain), colon, endometrium, skin, prostate, thyroid, and other types of cancer), immune system conditions (e.g., HIV infection and/or associated acquired immune deficiencies), endocrine conditions (e.g., diabetes), neurodegenerative diseases (e.g., Parkinson's disease, Alzheimer's disease, etc.), and any other medical condition for which a cell-based therapy is available or desirable.

The ability to optically analyze sorted cells using an imaging system enables combining the analysis of the cells that takes place upstream of the sorting means, which is carried out based on optical properties that can be measured using a flow cytometer and which is used in the sorting decision, and the imaging analysis. The imaging analysis enables further identification and characterization of the sorted cells. This additional information can be used to confirm the properties of the cells and access the suitability of the sorted cells for specific applications, such as for therapeutic uses. If the sorted cells sufficiently meet the criteria for use in the therapeutic application of interest, the cells may be released from the flow-through chamber by reversing the immobilization, as described above. For example, the cells may be collected into a suitable collection vessel (e.g., a sterile collection vessel with appropriate medium), transplanted into a patient according to a transplantation protocol, or stored for later transplantation, e.g., stored frozen in a suitable freezing medium. If the sorted cells fail to meet the suitability requirements, the cells can be released from the flow-through chamber and discarded.

Thus, in some embodiments, the invention provides fluidic sorting systems that include a flow-through chamber configured to receive a particle (e.g., a cell) in a fluid stream from a catcher tube, and a particle immobilization component configured to reversibly immobilize the particle within the flow-through chamber. The fluidic sorting system is a closed fluidic sorting system, e.g., a system in which a movable catcher tube disposed downstream of the interrogation point moves to the core stream when a particle of interest (e.g., a particle meeting certain spectral, size, granularity, or other criteria) is detected at the interrogation point, such that the catcher tube captures the particle of interest downstream of the interrogation point. In certain embodiments, the particle immobilization component reversibly immobilizes the particle via magnetic means. For example, the particle immobilization component optionally includes a magnetic field generator, such as a permanent magnet, an electromagnet, or both. Accordingly, fluidic sorting systems of the invention may include magnetically-labeled particles. In other embodiments, immobilization is achieved via acoustic means, e.g., in which the particle immobilization component includes an acoustic field generator.

Optionally, fluidic sorting systems of the invention further include an analysis component configured to analyze the immobilized particle within the flow-through chamber. In certain aspects, the analysis component includes a microscope, e.g., a conventional microscope, a fluorescence microscope, a confocal microscope, a laser scanning microscope, or any other microscope useful for analyzing one or more particle features of interest. To facilitate observation of the immobilized particle, the flow-through chamber optionally includes a flow-through optical cuvette.

The present invention also provides methods that include catching a particle (e.g., a cell) flowing from an interrogation point in a fluidic sorting system, delivering the particle to a flow-through chamber, and immobilizing, preferably in a reversible manner, the particle within the flow-through chamber. In some aspects, immobilizing the particle optionally includes generating a magnetic field within the flow-through chamber. In other aspects, immobilizing the particle comprises generating an acoustic field within the flow-through chamber.

According to one embodiment, the methods further include analyzing the immobilized particle within the flow-through chamber. Analyzing the immobilized particle may include observing the particle using a microscope, e.g., a conventional microscope, a fluorescence microscope, a confocal microscope, a laser scanning microscope, and the like. Optionally, the methods include reversing the immobilization to release the particle from the flow-through chamber and collecting the released particle.

In other aspects, components for use in a fluidic sorting system are provided. The components include a flow-through chamber configured to receive a particle in a fluid stream from a catcher tube, and an immobilization subcomponent configured to reversibly immobilize the particle within the flow-through chamber cell. The immobilization subcomponent may include a magnetic field generator (e.g., a permanent magnet and/or an electromagnet) or an acoustic field generator. According to one embodiment, the component further includes an analysis subcomponent configured to analyze the particle immobilized within the flow-through chamber. The analysis component optionally includes a microscope, such as a conventional microscope, a fluorescence microscope, a confocal microscope, a laser scanning microscope, and so forth. In certain aspects, the flow-through chamber of components of the invention includes a flow-through optical cuvette. According to one embodiment not covered by the claimed invention, kits are provided that include a flow-through chamber configured to be fluidically coupled to a catcher tube in a fluidic sorting system, and an immobilization component configured to reversibly immobilize a particle within the flow-through chamber. The immobilization component optionally includes a magnetic field generator or an acoustic field generator.

The fluidic sorting systems, methods, components and kits summarized above are described in greater detail below.

### BRIEF DESCRIPTION OF THE FIGURES

FIG. 1 provides a schematic view of a fluidic sorting system in accordance with an embodiment of the invention.
FIG. 2 is a schematic illustration of a fluidic sorting system in accordance with another embodiment of the invention.

### DETAILED DESCRIPTION

In further describing embodiments of the invention, aspects of embodiments of the fluidic sorting systems will be described first in greater detail. Thereafter, aspects of embodiments of the methods of using the fluidic sorting systems are described in greater detail. Next, aspects of embodiments of the components and kits not covered by the claimed invention are described in greater detail.

### SYSTEMS

### Sorting

As used herein, a closed-fluidic sorter refers to a sorter, such as a sorting flow cytometer, that sorts particles suspended in a fluid stream passing through a flow channel by redirecting a portion of the fluid stream containing a particle of interest into a separate fluidic channel, without ejecting the flow stream through a nozzle in an in-air droplet stream. Particles of interest are identified by analyzing the particles as they flow through a detection region (interrogation point) within the flow channel, as with a typical sorting flow cytometer. The temporary redirection of the fluid stream preferably is carried out using a moveable catcher tube as described in U.S. Patent No. 5,030,002. Alternatively, the temporary redirection of the fluid stream can be carried out using any valve mechanism that can effectively redirect the fluid stream under automated control in response to the detection of a particle of interest.

As in a typical sorting flow cytometer, particles suspended in a liquid medium are passed through a narrow fluidic channel one at a time past a detection region. Particles are labeled prior to analysis with one or more fluorescent dyes to facilitate identification. While passing the detection region, labeled particles are exposed to excitation light, typically from one or more lasers, and the resulting particle fluorescence is measured. Typically, the amount of excitation light scattered by the particles also is measured. The amount of scattered light and the intensity of emitted fluorescent light from each of the bound labels provide a characterization of the labeled particles. Typically, particles to be sorted (i.e., particles of interest) are identified using some criterion that is based on one or more of the optical properties.

### Flow-through Chamber

The fluidic sorting systems of the invention include a flow-through chamber configured to receive a particle in a fluid stream from a catcher tube and are configured to immobilize the particle in the flow-through chamber. Optionally, the systems are configured to analyze the immobilized particles in the flow-through chamber. The flow-through chamber may have any convenient configuration and be fabricated from any convenient material. In one aspect, the flow-through chamber is a flow-through optical cuvette, e.g., a cuvette of sufficient optical quality that particles within the cuvette may be analyzed, e.g., using a microscope system. The dimensions of the flow-through chamber may vary. Dimensions of the flow-through chamber may be chosen according to, e.g., the desired fluid volume to be accommodated by the flow-through chamber, etc. Flow-through chambers typically will have a volume of from about one to greater than 10 milliliters. Flow-through chambers of interest include at least one fluid entry port and at least one fluid exit port, where the at least one entry and exit ports are configured to provide for fluid flow into, through, and out of the flow-through chamber. Flow-through chambers may include additional features, where desired, such as multiple ports, valves, etc. Flow-through chambers as employed in systems of the invention may be fabricated using any convenient protocol, such as via microfluidic device fabrication protocols.

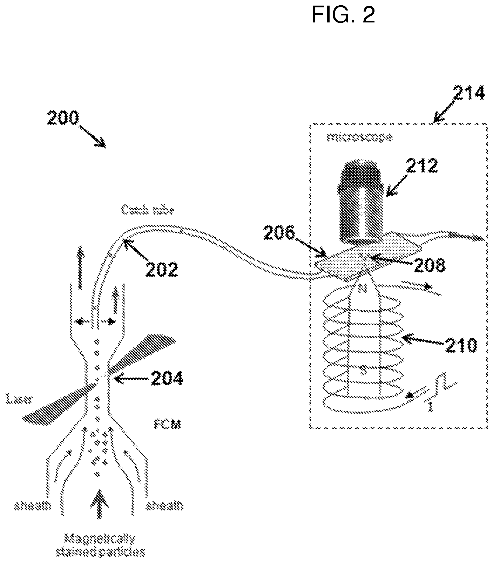

In embodiments in which sorting is carried out using a moving catcher tube, as described in, e.g., U.S. Patent No. 5,030,002, the flow-through chamber of the present invention is fluidically coupled to the moving catcher tube and configured to receive a particle of interest selectively caught by the catcher tube, as schematically illustrated in Figure 2.

### Immobilization

Operatively coupled to the flow-through chamber in systems of the invention is a particle immobilization component. By particle immobilization component, it is meant a component of a fluidic sorting system configured to immobilize, preferably reversibly, a particle within a flow-through chamber of the system. The particle immobilization subcomponent may be configured to immobilize a particle of interest by a variety of mechanisms.

In some embodiments, the particle immobilization subcomponent may include a magnetic field generator, e.g., a permanent magnet, an electromagnet, or a combination thereof. For use with a magnetic immobilization subcomponent, particles are labeled with one or more magnetic beads prior to analysis. This magnetic labeling is addition to any fluorescent labeling, if any, used to identify cells of interest. Typically, cells will be labeled with both magnetically and fluorescently labeled antibodies, although in some case, sorting of particles of interest may be based solely on light scatter properties or on inherent cellular fluorescence.

Magnetic labeling of cells typically is carried out using magnetic beads conjugated to one or more antibodies that bind to a polypeptide on the surface of the cell, e.g., a cell surface receptor. For example, white blood cells may be labeled with magnetic beads that include antibodies that specifically bind to CD45, stem cells may be labeled with magnetic beads that include antibodies that specifically bind to CD34, and so forth. Antibodies bound to magnetic beads suitable for labeling cells are well known in the art and are commercially available from multiple vendors, e.g., BD Biosciences (San Jose, CA). When the magnetically labeled particle enters the flow-through chamber, a magnetic field generated by the particle immobilization subcomponent immobilizes the particle within the flow-through chamber.

Particle immobilization is reversible. For example, when the particle immobilization subcomponent includes a permanent magnet, the magnet may be positioned sufficiently adjacent to the flow-through chamber such that a magnetic field generated by the magnet immobilizes the particle. When particle immobilization is no longer desired, e.g., when it is desirable to collect the one or more immobilized particles, the distance between the permanent magnet and the flow-through chamber may be increased, e.g., by moving the magnet to a location more distant to the flow-through chamber such that the magnetic field is insufficient to maintain immobilization of the particle. In a related aspect, when the particle immobilization subcomponent includes an electromagnet positioned adjacent to the flow-through chamber, a current may be allowed to flow through a coil of the magnet, thereby generating a magnetic field sufficient to immobilize the particles in the flow-through chamber. Reversing the immobilization may be achieved by reducing or eliminating the current flowing through the coil. Also provided by the present invention are subcomponents for the reversible immobilization of particles that include one of a permanent magnet and an electromagnet for reversibly immobilizing magnetically labeled particles within the flow-through chamber.

In another embodiment, particle immobilization is achieved using a particle immobilization subcomponent that includes an acoustic field and/or pressure node generator configured to produce an acoustic or displacement field within the flow-through chamber. As used herein, an "acoustic field generator" includes a vibration generator such as a piezoelectric transducer (which converts electrical energy into mechanical energy), a displacement generator, an in-line drive element, or any other device capable of producing within the flow-through chamber an acoustic or displacement field sufficient to immobilize the particle of interest, e.g., such that the pressure from the acoustic or displacement field traps the particle of interest at a surface of the flow-through chamber. Acoustic immobilization and/or concentration of particles within a fluidic device are described in, e.g., U.S. Patent No. 7,837,040, entitled: "Acoustic Concentration of Particles in Fluid Flow", and U.S. Patent No. 7,373,805, entitled: "Apparatus for Directing Particles in a Fluid".

Particle immobilization using an acoustic field generator and/or pressure node generator is reversible. Reversing immobilization of the particle within the flow-through chamber is achieved by reducing the intensity of (or eliminating altogether) the acoustic or displacement field generated by the acoustic field generator, e.g., by switching the generator off.

In certain aspects, the particle immobilization subcomponent is synchronized with the flow-through chamber. For example, the flow-through chamber may be connected to multiple ports with associated valves. The valves may be synchronized with a switch (e.g., a switch that reduces or eliminates magnetic field generation by the particle immobilization subcomponent) that reverses immobilization, such that the desired cells may be collected in one port for further processing, and the undesired cells discarded.

### Analysis component

Systems of the invention optionally include an analysis component configured to analyze an immobilized particle within the flow-through chamber. The analysis component may include any device capable of analyzing one or more particle features of interest to a user of the system. According to this embodiment, the analysis component is operatively coupled (e.g., optically coupled) to the flow-through chamber to permit analysis of particles immobilized therein.

In certain aspects, the analysis component includes a microscope. The microscope may be a conventional microscope (e.g., a microscope that illuminates the particle with light to produce a magnified image of the particle), a fluorescence microscope, a confocal microscope, a laser scanning microscope, or any other type of microscope useful for analyzing particles in a fluidic sorting system. According to this embodiment, the microscope is optically coupled to the flow-through chamber. For example, the microscope and flow-through chamber can be configured in such a way that the microscope illuminates (e.g., provides excitation radiation to) the immobilized particle and generates a magnified image of the particle.

Traditional flow cytometric systems analyze particles according to size, granularity, and fluorescence emission. The optional analysis component of the present invention enables direct observation of particle attributes that cannot be observed using traditional flow cytometric systems. For example, when the analysis subcomponent includes a microscope and the particle is a cell, the subcellular physical, chemical and/or biological properties of the immobilized cell can be observed and/or measured.

The particle collected by the catcher tube may subsequently be delivered to an appropriate collection vessel, e.g., a collection tube, a microwell, a tissue culture plate, or any collection vessel convenient for a further application of interest, e.g., nucleic acid isolation, cell and/or tissue culture, and the like. Collection and deposition of a cell of interest may be carried out under sterile conditions for applications adversely affected by, e.g., microbial contamination.

### Description based on the figures

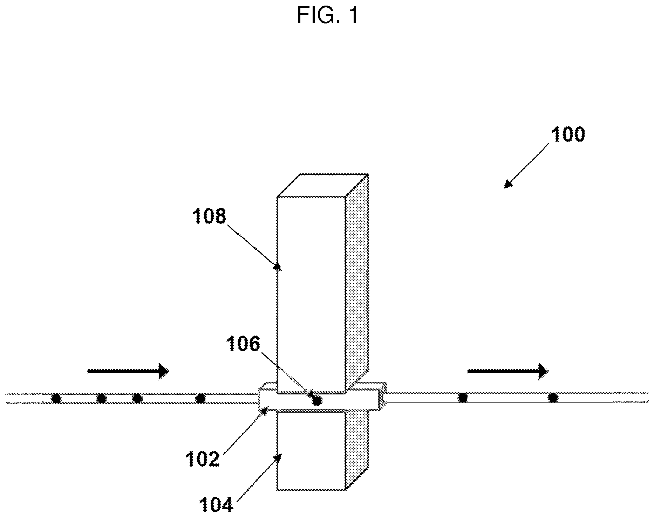

A schematic view of a fluidic sorting system in accordance with an embodiment of the invention is provided in **FIG. 1****.** As shown, system **100** includes flow-through chamber **102** configured to receive a particle in a fluid stream from a catcher tube (shown as solid black circles). The system also includes particle immobilization subcomponent **104** configured to reversibly immobilize particle **106** within the flow-through chamber. Because it may be desirable to analyze the particle in the flow-through chamber, the system optionally includes analysis subcomponent **108** configured to analyze the immobilized particle within flow-through chamber.

A fluidic sorting system according to a second embodiment of the invention is schematically illustrated in **FIG. 2****.** In this embodiment, fluidic sorting system **200** is a closed fluidic sorting system having moving catcher tube **202** configured to receive one or more particles of interest as the particles flow from interrogation point **204.** Particles collected by catcher tube **202** are delivered to a flow-through chamber, e.g., flow-through optical cuvette **206** configured to receive a particle in a fluid stream from the catcher tube. Magnetically labeled particles of interest **208** are immobilized within cuvette **206** by a magnetic field generated by a particle immobilization component that includes electromagnet **210.** Optionally, the system includes an analysis component that includes microscope **212** configured to analyze one or more particles, e.g., particles **208,** immobilized within flow-through chamber **206.** In one aspect, the present invention provides the system schematically illustrated in FIG. 2 as a complete system, e.g., a closed fluidic sorter with integrated flow-through chamber and a component for reversible immobilization (and optionally components for analysis and/or collection of a particle of interest). In other embodiments, as described further below, but not covered by the claimed invention, the flow-through chamber and immobilization component (and optionally an analysis component and/or collection means) may also be provided as a component, or as a kit, e.g., for providing additional functionality to an existing fluidic sorting system. Components and kits are described herein below.

According to certain embodiments of the present invention, e.g., fluidic sorting system **200** schematically illustrated in Figure 2, reversible particle immobilization provided by the present invention is useful not only for permitting observation of particles of interest in the flow-through chamber, but also for analyzing and collecting particles of interest (e.g., rare cells) at particle densities not achievable using conventional closed-fluidic sorters. As discussed above, when the catcher tube of a conventional closed fluidic sorter is not selectively capturing a particle of interest, the catcher tube is collecting sheath fluid. Accordingly, the particles of interest are collected at densities too low for certain types of analyses. As provided by certain embodiments of fluidic sorting systems of the present invention, immobilization of particles downstream of the catcher tube effectively concentrates the particles, facilitating their efficient analysis within the system and/or their ultimate collection at densities sufficient for subsequent analysis, experimentation, manipulation and/or use.

### METHODS

Methods, e.g., methods of using the fluidic sorting systems described above, are also provided by the invention. The methods include sorting a particle of interest using a closed-fluidic sorting system, delivering the particle to a flow-through chamber, and immobilizing, preferably reversibly, the particle within a flow-through chamber.

In one embodiment of the methods of the invention, the particles are immobilized using a magnetic field generated within the flow-through chamber, as described above. In other embodiments, the particles are immobilized using an acoustic field and/or pressure node within the flow-through chamber, as described above.

In other embodiments, the methods further include analyzing the immobilized particle within the flow-through chamber. Analyzing the immobilized particle preferably is carried out using an optical microscope. The microscope may be any microscope that finds use in analyzing particle features of interest, including a conventional microscope, a fluorescence microscope, a confocal microscope, or a laser scanning microscope.

It may be desirable to collect the particle from the flow-through chamber to permit subsequent analysis, experimentation, manipulation and/or use of the particle. As such, methods of the invention optionally include reversing the immobilization to release the particle and collecting the released particle. Suitable approaches for collecting the released particle include employing a particle sorter operatively coupled to the flow-through chamber.

### KITS

The present disclosure also provides kits not covered by the claimed invention. The kits are useful, e.g., for incorporating the flow-through chamber and immobilization component described above into a fluidic sorting system lacking such components. Kits include a flow-through chamber (e.g., a flow-through optical cuvette) configured to be fluidically coupled to a fluidic sorting system, and an immobilization component configured to reversibly immobilize a particle within the flow-through chamber. Optionally, the kits include an analysis component configured to analyze a reversibly immobilized particle within the flow-through chamber. The immobilization component may include a magnetic field generator or an acoustic field generator, as described above. The optional analysis component may include a microscope, e.g., a conventional microscope, a fluorescence microscope, a confocal microscope, a laser scanning microscope, and any other type of microscope that finds use in fluidic sorting systems.

The kit components (including the flow-through chamber) may be provided as a single unit (e.g., such as component **214** in Figure 2), where incorporating the components into the fluidic sorting system could be readily accomplished by fluidically coupling the flow-through chamber of the unit to a fluidic sorting system, e.g., a closed fluidic sorter. Alternatively, one or more of, e.g., the flow-through chamber and/or the immobilization component may be provided as individual kit components for separate integration into a fluidic sorting system.

The subject kits will further include instructions for, e.g., operatively coupling the kit components (either as a single unit or individually) to a fluidic sorting system. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, etc., on which the information has been recorded. Yet another means that may be present is a website address which may be used via the internet to access the information at a removed site. Any convenient means may be present in the kits.

It is to be understood that this invention is not limited to particular embodiments described herein, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments are specifically embraced by the present invention and are disclosed herein just as if each and every combination was individually and explicitly disclosed, to the extent that such combinations embrace operable processes and/or devices/systems/kits. In addition, all sub-combinations listed in the embodiments describing such variables are also specifically embraced by the present invention and are disclosed herein just as if each and every such sub-combination of chemical groups was individually and explicitly disclosed herein.

As will be apparent to those of skill in the art upon reading this disclosure, each of the individual embodiments described and illustrated herein has discrete components and features which may be readily separated from or combined with the features of any of the other several embodiments without departing from the scope of the present invention as defined by the appended claims.

Any recited method can be carried out in the order of events recited or in any other order which is logically possible.

## Claims

1. A closed- fluidic sorting system (200) comprising:
a detection component configured to detect a particle of interest (106, 208) in a particle flow stream at an interrogation point (204); and
a movable catcher tube (202) configured to be activated to move into a position to receive the detected particle of interest (106, 208) flowing from the interrogation point (204) when the particle of interest (106, 208) is detected at the interrogation point (204)
a flow-through chamber (102, 206) configured to receive the particle (106, 208) from the movable catcher tube (202), wherein the flow-through chamber (102, 206) consists of a single fluid entry port, a single fluid exit port and a particle immobilization component (104, 210) positioned therebetween;
wherein the particle immobilization component (104, 210) (104) is configured to reversibly immobilize the particle (106, 208) within the flow-through chamber (102, 206).

2. The closed- fluidic sorting system (200) according to claim 1, wherein the particle immobilization component (104, 210) comprises a magnetic field generator, which preferably comprises a permanent magnet, and/or an electromagnet (210).

3. The closed- fluidic sorting system (200) according to any of the preceding claims, further comprising an analysis component (108, 212) configured to analyze the immobilized particle (106, 208) within the flow-through chamber (102, 206) preferably wherein the analysis component comprises a microscope (212), more preferably wherein the microscope (212) is selected from the group consisting of: a conventional microscope, a fluorescence microscope, a confocal microscope, and a laser scanning microscope.

4. The closed- fluidic sorting system (200) according to any of the preceding claims, wherein the flow-through chamber (102, 206) is configured to receive a particle (106, 208) that is a cell.

5. The closed- fluidic sorting system (200) according to any of the preceding claims, wherein the flow-through chamber comprises a flow-through optical cuvette (206).

6. The closed- fluidic sorting system (200) according to any of the preceding claims, wherein the closed-fluidic sorting system (200) comprises magnetically-labeled particles (208).

7. The closed- fluidic sorting system (200) according to any of the preceding claims, wherein the closed-fluidic sorting system (200) further comprises a particle sorter configured to receive the particle from the flow-through chamber, preferably wherein the particle sorter comprises a catcher tube configured to receive the particle from the flow-through chamber when the catcher tube is activated.

8. A method comprising:
(a) detecting a particle of interest in a particle flow stream at an interrogation point (204);
(b) redirecting the detected particle of interest into a movable catcher tube (202) separate from the fluid of the particle flow stream;
(c) catching the particle of interest (106, 208) flowing from the interrogation point (204) in said emovable catcher tube (202), wherein the movable catcher tube (202) is configured to be activated to move into a position to receive the detected particle of interest (106, 208) when the particle of interest (106, 208) is detected at the interrogation point (204);
(d) delivering the particle of interest (106, 208) to a flow-through chamber (102, 206) fluidically coupled to the catcher tube (202), wherein the flow-through chamber (102, 206) consists of a single fluid entry port, a single fluid exit port and a particle immobilization component (104, 210) positioned therebetween, wherein the particle immobilization component (104, 210) is configured to reversibly immobilize the particle of interest (106, 208) within the flow-through chamber (102, 206); and
(e) reversibly immobilizing the particle of interest (106, 208) within the flow-through chamber (102, 206).

9. The method according to claim 8, wherein reversibly immobilizing the particle of interest (106, 208) comprises generating a magnetic field within the flow-through chamber (102, 206).

10. The method according to claim 9 or 10, further comprising analyzing the immobilized particle of interest (106, 208) within the flow-through chamber (102, 206), preferably wherein analyzing the immobilized particle of interest (106, 208) comprises observing the particle of interest (106, 208) using a microscope (212), more preferably wherein the particle of interest (106, 208) is observed using a microscope (212) selected from: a conventional microscope, a fluorescence microscope, a confocal microscope, and a laser scanning microscope.

11. The method according to any of the preceding claims 8-10, further comprising reversing the immobilization to release the particle of interest (106, 208) and collecting the released particle. particle of interest (106, 208).

12. The method according to any of the preceding claims 8-11, wherein the particle of interest (106, 208) is a cell.

## Patentansprüche

1. Geschlossenes fluidisches Sortiersystem (200), umfassend:
eine Festellungskomponente, die so konfiguriert ist, dass sie ein Partikel von Interesse (106, 208) in einem Partikelflussstrom an einem Abfragepunkt (204) feststellt; und
ein bewegliches Auffangrohr (202), das so konfiguriert ist, dass es aktiviert wird, um sich in eine Position zu bewegen, in der es das festgestellte Partikel von Interesse (106, 208), das vom Abfragepunkt (204) fließt, empfängt, wenn das Partikel von Interesse (106, 208) am Abfragepunkt (204) festgestellt wird;
eine Durchflusskammer (102, 206), die so konfiguriert ist, dass sie das Partikel (106, 208) aus dem beweglichen Auffangrohr (202) empfängt, wobei die Durchflusskammer (102, 206) aus einem einzelnen Fluideinlassanschluss, einem einzelnen Fluidauslassanschluss und einer dazwischen positionierten Partikelimmobilisierungskomponente (104, 210) besteht;
wobei die Partikelimmobilisierungskomponente (104, 210) so konfiguriert ist, dass sie das Partikel (106, 208) innerhalb der Durchflusskammer (102, 206) reversibel immobilisiert.

2. Geschlossenes fluidisches Sortiersystem (200) nach Anspruch 1, wobei die Partikelimmobilisierungskomponente (104, 210) einen Magnetfeldgenerator umfasst, der vorzugsweise einen Permanentmagneten und/oder einen Elektromagneten (210) umfasst.

3. Geschlossenes fluidisches Sortiersystem (200) nach einem der vorstehenden Ansprüche, ferner umfassend eine Analysekomponente (108, 212), die so konfiguriert ist, dass sie die immobilisierten Partikel (106, 208) in der Durchflusskammer (102, 206) analysiert, wobei die Analysekomponente vorzugsweise ein Mikroskop (212) umfasst, wobei das Mikroskop (212) vorzugsweise aus der Gruppe ausgewählt ist, die aus Folgendem besteht: einem herkömmlichen Mikroskop, einem Fluoreszenzmikroskop, einem konfokalen Mikroskop und einem Laser-Scanning-Mikroskop.

4. Geschlossenes fluidisches Sortiersystem (200) nach einem der vorstehenden Ansprüche, wobei die Durchflusskammer (102, 206) so konfiguriert ist, dass sie ein Partikel (106, 208) empfängt, das eine Zelle ist.

5. Geschlossenes fluidisches Sortiersystem (200) nach einem der vorstehenden Ansprüche, wobei die Durchflusskammer eine optische Durchflussküvette (206) umfasst.

6. Geschlossenes fluidisches Sortiersystem (200) nach einem der vorstehenden Ansprüche, wobei das geschlossene fluidische Sortiersystem (200) magnetisch gekennzeichnete Partikel (208) umfasst.

7. Geschlossenes fluidisches Sortiersystem (200) nach einem der vorstehenden Ansprüche, wobei das geschlossene fluidische Sortiersystem (200) ferner einen Partikelsortierer umfasst, der so konfiguriert ist, dass er das Partikel aus der Durchflusskammer empfängt, vorzugsweise wobei der Partikelsortierer ein Auffangrohr umfasst, das so konfiguriert ist, dass es das Partikel aus der Durchflusskammer empfängt, wenn das Auffangrohr aktiviert ist.

8. Verfahren, umfassend:
(a) Feststellen eines Partikels von Interesse in einem Partikelflussstrom an einem Abfragepunkt (204);
(b) Umleiten des festgestellten Partikels von Interesse in ein bewegliches Auffangrohr (202), das vom Fluid des Partikelflussstroms getrennt ist;
(c) Auffangen des von dem Abfragepunkt (204) fließenden Partikels von Interesse (106, 208) in dem beweglichen Auffangrohr (202), wobei das bewegliche Auffangrohr (202) so konfiguriert ist, dass es aktiviert wird, um sich in eine Position zu bewegen, um das festgestellte Partikel von Interesse (106, 208) zu empfangen, wenn das Partikel von Interesse (106, 208) an dem Abfragepunkt (204) festgestellt wird;
(d) Zuführen des Partikels von Interesse (106, 208) zu einer Durchflusskammer (102, 206), die fluidisch mit dem Auffangrohr (202) gekoppelt ist, wobei die Durchflusskammer (102, 206) aus einem einzelnen Fluideinlassanschluss, einem einzelnen Fluidauslassanschluss und einer dazwischen positionierten Partikelimmobilisierungskomponente (104, 210) besteht, wobei die Partikelimmobilisierungskomponente (104, 210) so konfiguriert ist, dass sie das Partikel von Interesse (106, 208) innerhalb der Durchflusskammer (102, 206) reversibel immobilisiert; und
(e) reversibles Immobilisieren des Partikels von Interesse (106, 208) innerhalb der Durchflusskammer (102, 206).

9. Verfahren nach Anspruch 8, wobei das reversible Immobilisieren des Partikels von Interesse (106, 208) das Erzeugen eines Magnetfeldes innerhalb der Durchflusskammer (102, 206) umfasst.

10. Verfahren nach Anspruch 9 oder 10, ferner umfassend Analysieren des immobilisierten Partikels von Interesse (106, 208) in der Durchflusskammer (102, 206), wobei das Analysieren des immobilisierten Partikels von Interesse (106, 208) das Beobachten des Partikels von Interesse (106, 208) unter Verwendung eines Mikroskops (212) umfasst, noch bevorzugter, wobei das Partikel von Interesse (106, 208) unter Verwendung eines Mikroskops (212) beobachtet wird, das aus Folgendem ausgewählt ist: einem herkömmlichen Mikroskop, einem Fluoreszenzmikroskop, einem konfokalen Mikroskop und einem Laser-Scanning-Mikroskop.

11. Verfahren nach einem der vorstehenden Ansprüche 8 bis 10, ferner umfassend das Umkehren der Immobilisierung zur Freisetzung des Partikels von Interesse (106, 208) und das Sammeln des freigesetzten Partikels von Interesse (106, 208).

12. Verfahren nach einem der vorstehenden Ansprüche 8 bis 11, wobei das Partikel von Interesse (106, 208) eine Zelle ist.

## Revendications

1. Système de tri fluidique fermé (200) comprenant :
un composant de détection conçu pour détecter une particule d'intérêt (106, 208) dans un flux d'écoulement de particules au niveau d'un point d'interrogation (204) ; et
un tube de récupération mobile (202) conçu pour être activé pour se déplacer dans une position pour recevoir la particule d'intérêt détectée (106, 208) s'écoulant depuis le point d'interrogation (204) lorsque la particule d'intérêt (106, 208) est détectée au niveau du point d'interrogation (204) ;
une chambre d'écoulement continu (102, 206) conçue pour recevoir la particule (106, 208) depuis le tube de récupération mobile (202), dans lequel la chambre d'écoulement continu (102, 206) consiste en un orifice d'entrée de fluide unique, un orifice de sortie de fluide unique et un composant d'immobilisation de particules (104, 210) positionné entre eux ;
dans lequel le composant d'immobilisation de particules (104, 210) est conçu pour immobiliser de manière réversible la particule (106, 208) à l'intérieur de la chambre d'écoulement continu (102, 206).

2. Système de tri fluidique fermé (200) selon la revendication 1, dans lequel le composant d'immobilisation de particules (104, 210) comprend un générateur de champ magnétique, qui comprend de préférence un aimant permanent, et/ou un électroaimant (210).

3. Système de tri fluidique fermé (200) selon l'une quelconque des revendications précédentes, comprenant en outre un composant d'analyse (108, 212) configuré pour analyser la particule immobilisée (106, 208) à l'intérieur de la chambre d'écoulement continu (102, 206), de préférence dans lequel le composant d'analyse comprend un microscope (212), plus préférablement dans lequel le microscope (212) est choisi dans le groupe constitué : d'un microscope classique, d'un microscope à fluorescence, d'un microscope confocal et d'un microscope à balayage laser.

4. Système de tri fluidique fermé (200) selon l'une quelconque des revendications précédentes, dans lequel la chambre d'écoulement continu (102, 206) est conçue pour recevoir une particule (106, 208) qui est une cellule.

5. Système de tri fluidique fermé (200) selon l'une quelconque des revendications précédentes, dans lequel la chambre d'écoulement continu comprend une cuvette optique d'écoulement continu (206).

6. Système de tri fluidique fermé (200) selon l'une quelconque des revendications précédentes, dans lequel le système de tri fluidique fermé (200) comprend des particules marquées magnétiquement (208).

7. Système de tri fluidique fermé (200) selon l'une quelconque des revendications précédentes, dans lequel le système de tri fluidique fermé (200) comprend en outre un trieur de particules conçu pour recevoir la particule depuis la chambre d'écoulement continu, de préférence dans lequel le trieur de particules comprend un tube de récupération conçu pour recevoir la particule depuis la chambre d'écoulement continu lorsque le tube de récupération est activé.

8. Procédé comprenant :
(a) la détection d'une particule d'intérêt dans un flux d'écoulement de particules au niveau d'un point d'interrogation (204) ;
(b) la redirection de la particule d'intérêt détectée dans un tube de récupération mobile (202) séparé du fluide du flux d'écoulement de particules ;
(c) le fait d'attraper la particule d'intérêt (106, 208) s'écoulant du point d'interrogation (204) dans ledit tube de récupération mobile (202), dans lequel le tube de récupération mobile (202) est conçu pour être activé pour se déplacer dans une position pour recevoir la particule d'intérêt détectée (106, 208) lorsque la particule d'intérêt (106, 208) est détectée au niveau du point d'interrogation (204) ;
(d) la distribution de la particule d'intérêt (106, 208) à une chambre d'écoulement continu (102, 206) accouplée fluidiquement au tube de récupération (202), dans lequel la chambre d'écoulement continu (102, 206) consiste en un orifice d'entrée de fluide unique, un orifice de sortie de fluide unique et un composant d'immobilisation de particule (104, 210) positionné entre eux, dans lequel le composant d'immobilisation de particule (104, 210) est conçu pour immobiliser de manière réversible la particule d'intérêt (106, 208) à l'intérieur de la chambre d'écoulement continu (102, 206) ; et
(e) l'immobilisation de manière réversible de la particule d'intérêt (106, 208) à l'intérieur de la chambre d'écoulement continu (102, 206).

9. Procédé selon la revendication 8, dans lequel l'immobilisation réversible de la particule d'intérêt (106, 208) comprend la génération d'un champ magnétique à l'intérieur de la chambre d'écoulement continu (102, 206).

10. Procédé selon la revendication 9 ou 10, comprenant en outre l'analyse de la particule d'intérêt immobilisée (106, 208) à l'intérieur de la chambre d'écoulement continu (102, 206), de préférence dans lequel l'analyse de la particule d'intérêt (106, 208) immobilisée comprend l'observation de la particule d'intérêt (106, 208) à l'aide d'un microscope (212), plus préférablement dans lequel la particule d'intérêt (106, 208) est observée à l'aide d'un microscope (212) choisi parmi : un microscope classique, un microscope à fluorescence, un microscope confocal et un microscope à balayage laser.

11. Procédé selon l'une quelconque des revendications 8 à 10 précédentes, comprenant en outre l'inversion de l'immobilisation pour libérer la particule d'intérêt (106, 208) et la collecte de la particule d'intérêt (106, 208) libérée.

12. Procédé selon l'une quelconque des revendications 8 à 11 précédentes, dans lequel la particule d'intérêt (106, 208) est une cellule.
